Europäisches Patentamt

European Patent Office

Office européen des brevets

(11) Publication number : **0 480 758 A1**

## (12) EUROPEAN PATENT APPLICATION

(21) Application number : **91309394.4**

(51) Int. Cl.[5] : **B01J 2/30, C07D 487/08**

(22) Date of filing : **11.10.91**

(30) Priority : **12.10.90 JP 272273/90**

(43) Date of publication of application :
**15.04.92 Bulletin 92/16**

(84) Designated Contracting States :
**AT BE CH DE DK ES FR GB GR IT LI LU NL SE**

(71) Applicant : **Tosoh Corporation**
**4560, Kaisei-cho**
**Shinnanyo-shi, Yamaguchi-ken (JP)**

(72) Inventor : **Onaka, Tadao**
**17-11, Nagato-cho**
**Shinnanyo-shi, Yamaguchi-ken (JP)**
Inventor : **Nomura, Akihiko**
**11-5, Nomura 1-chome**
**Shinnanyo-shi, Yamaguchi-ken (JP)**
Inventor : **Fujii, Masahiko**
**840-1, Oazashimogami**
**Tokuyama-shi, Yamaguchi-ken (JP)**

(74) Representative : **Kearney, Kevin David**
**Nicholas et al**
**KILBURN & STRODE 30 John Street**
**London, WC1N 2DD (GB)**

(54) **Granular or particulate triethylenediamine and process for its production.**

(57)    Triethylenediamine which is a granular or particulate powder of at least 30 mesh.

EP 0 480 758 A1

Jouve, 18, rue Saint-Denis, 75001 PARIS

The present invention relates to granular or particulate triethylenediamine (hereinafter referred to simply as TEDA) which has excellent flowability and storage stability and which can readily be dispersed and dissolved in a solution, and a process for its production.

TEDA is a compound which is highly sublimable and has strong coherence and adherence and which is highly hygroscopic and agglomerative. It readily undergoes agglomeration due to inclusion of a small amount of moisture or due to a temperature rise. Therefore, such TEDA powder is required to be handled with due care, and once such a powder has agglomerated, handling tends to be extremely difficult.

In processes for handling powder, it is common to employ various measures to prevent clogging due to formation of bridging. For example, there may be mentioned improvements in the shape, capacity and material of the container for storage, or as a method of withdrawing the powder, a method may be employed in which a vibrating machine, an air knocker or a compressed air jetting apparatus is used.

However, TEDA powder is susceptible to agglomeration due to the highly sublimable and hygroscopic nature in addition to the formation of bridging due to a physical phenomenon, and thus, no adequate effect has been obtained by such measures depending solely on the equipments used for the process.

As a measure to prevent agglomeration of such an agglomerative powder, it is common to employ a method of removing impurities contained in the powder, a method of adding an anti-agglomeration agent to the powder, or a method of storing the powder by means of a closed vessel, since it can not be stored in a usual storage tank. However, TEDA powder undergoes agglomeration even when stored in a closed vessel. Further, highly sublimable TEDA has an increased tendency to agglomerate when the purity is increased. The conventional method of adding an anti-agglomeration agent, does not provide adequate effects. Thus, there has been no appropriate method for preventing agglomeration of TEDA powder.

Powders usually have coherence and adherence in many cases, and various measures have been taken to reduce such nature. However, in the case of agglomerative TEDA which is highly sublimable, sublimation and condensation are repeated due to a change of e.g. the external temperature, whereby a strong bridge will be formed between powder particles (crystals). TEDA will thereby agglomerate entirely in the container and tends to be difficult to disintegrate, whereby a mechanical means such as a vibrator will be practically useless. Further, once such a product has agglomerated in a container, it will be difficult to take out the product. Besides, a block of TEDA has extremely poor solubility, and it takes a long time to dissolve it.

TEDA is usually synthesized or produced from e.g. N-aminoethylpiperazine or hydroxyethylpiperazine. By such a method, TEDA is obtainable as slightly yellow white crystals. Such TEDA contains, as a by-product, an alkylpiperazine or the like. This by-product has an anti-agglomerating action to some extent. However, TEDA crystals of high purrity have been desired in recent years. Consequently, TEDA crystals having a higher purity are now produced as a result of improvements in the purification technique. Accordingly, the agglomerating nature of TEDA has been thereby sharply increased, and there has been a problem from the viewpoint of production processes or storage.

It is an object of the present invention to provide granular or particulate TEDA which has excellent flowability and storage stability and which can be readily dispersed and dissolved in a solution, and a method for producing it.

As a result of an extensive study in view of the above-mentioned circumstances, the present inventors have found it possible to effectively produce granular or particulate TEDA which has excellent flowability and storage stability and which can be readily dispersed and dissolved in a solution, by mixing from 3 to 20 parts by weight of water or an organic solvent and from 0.5 to 10 parts by weight of an additive to 100 parts by weight of powdery TEDA, followed by granulation and drying. The present invention has been accomplished on the basis of this discovery.

Thus, the present invention provides triethylenediamine which is a granular or particulate powder of at least 30 mesh (US Sieve Series, which has openings 590 micrometres across) preferably up to 10 mesh (US Sieve Series which has openings 2000 micrometres across) or more broadly up to 3.5 mesh (US Sieve Series which has openings 5660 micrometres across).

Further, the present invention provides a process for producing granular or particulate triethylene-diamine, which comprises mixing from 3 to 20 parts by weight of water or an organic solvent and from 0.5 to 10 parts by weight of an additive to 100 parts by weight of powdery triethylenediamine, followed by granulation and drying.

Now, the present invention will be described in detail with reference to the preferred embodiments.

The additive in the present invention includes celluloses such as hydroxypropyl cellulose, hydroxypropylmethyl cellulose, ethyl cellulose, methyl cellulose and carboxymethyl cellulose, polyvinyl pyrrolidone, polyvinyl alcohol, glycols, and polyethylene glycols.

The amount of such an additive to be incorporated, varies depending upon its ability as a binder. It is usually within a range of from 0.5 to 10 parts by weight, preferably from 1 to 5 parts by weight, per 100 parts by weight of TEDA. However, an excessive addition is undesirable, since it induces an excessive blocking phenomenon even in the granulation step and the drying step.

There is no particular restriction as to the solvent to be used for granulation, so long as it is capable of dissolving the additive. However, it is common to employ water or an alcohol such as methanol, ethanol or propanol. Preferred is methanol or ethanol in view of its efficiency in the drying step. The amount of such a solvent varies depending upon the type of the additive. However, it is usually within a range of from 3 to 20 parts by weight, preferably from 5 to 10 parts by weight, per 100 parts by weight of TEDA. Such an amount of the solvent is influential over the grain size of the product, and it is also an important condition for granulation. For example, if the amount incorporated is small, the solvent tends to hardly spread uniformly, whereby granulaticn tends to be difficult. On the other hand, if the amount is excessive, efficiency in the drying step tends to deteriorate.

In the granulation step, a mixing granulator is used. The particle size of the product is usually at least 30 mesh, preferably from 10 to 15 mesh. If the particle size is less than 30 mesh, no adequate flowability or storage stability will be obtained. Further, if particles or granules of less than 30 mesh are contained in a substantial amount, or if the particle size distribution becomes too wide, the blocking phenomenon of the powder tends to be promoted. In such a case, it is necessary to conduct a classification operation. The granulation time is usually from 1 to 10 minutes. The longer the granulation time, the larger the particle size, but the wider the particle size distribution.

Thus, the present invention provides an epoch-making technique which provides granular or particulate TEDA which is excellent in the flowability and storage stability and which shows excellent solubiltiy by mixing water or an organic solvent and an additive in a prescribed proportion to powdery TEDA, and a process for its production.

Now, the present invention will be described in further detail with reference to Examples. However, it should be understood that the present invention is by no means restricted by such specific Examples.

With respect to the obtained granular and particulate TEDA, the storage stability and the solubility were evaluated.

As an index for the storage stability, the agglomeration degree was measured in accordance with the following method. The measuring method and the evaluation standards were as follows.

A sample was packed in a container having a size of 5 cm x 5 cm and a height of 2 cm, and a plastic plate of 5 cm x 5 cm was placed thereon. A weight of 300 g was placed thereon, and the container was stored in a desiccater having a humidity of not higher than 1%. During the storage, the pressure exerted on the crystals was 12 g/cm$^2$. After storage in the desiccater for one month, the weight and the container were removed, and pressure was exerted to the center portion of the crystal block having the plastic plate located beneath, by a Kiya-type hardness meter, whereby the pressure at breakage was read. The values thus obtained were classified into the following three rankings, which were used as indices for evaluation of the agglomeration degree.

A rank: Crystal block which can readily be broken with a slight impact with a breaking pressure of not higher than 1.0 kg/cm$^2$ and in which no substantial progress of agglomeration was observed.

B rank: Crystal block with a breaking pressure of not higher than 10.0 kg/cm$^2$ which can not be broken by a low level of impact and in which agglomeration was found progressed entirely.

C rank: Crystal block which requires a considerably strong impact for breakage with a breaking pressure of at least 10.0 kg/cm$^2$ and in which agglomeration was found completely progressed.

Further, for an index of the solubility, a sample prepared in the same manner as for the measurement of the degree of agglomeration, was used, and it was put into 500 m$\ell$ of dipropylene glycol stirred at 50°C under a constant condition, whereby the dissolving time was measured.

EXAMPLE 1

3 kg of TEDA powder having a particle size of from 50 to 200 mesh (US Sieve Series which have openings 297 to 74 micrometres across) was put in a mixing granulator and while thoroughly stirring it, a solution containing 10 g of hydroxypropyl cellulose in 300 m$\ell$ of methanol, was added. In one minute after the addition, granular TEDA of from 10 to 30 mesh (US Sieve Series which have openings 2000 to 590 micrometres across) was obtained. However, this TEDA was in a wet state. Therefore, it was dried at 40°C for 30 minutes in a fluidized bed dryer.

The agglomeration degree of the dried powder was measured and found to be A rank. Namely, the powder underwent no agglomeration and exhibited high flowability. Further, in the measurement of the solubility, the sample was readily dispersed and dissolved in the solution, and the dissolving time was very short at a level of 6 minutes.

Thus, the granular TEDA showed excellent storage stability and solubility.

COMPARATIVE EXEMPLE 1

TEDA powder having a particle size of from 50 to 200 mesh was subjected to the measurement of the agglomeration degree and the solubility in the same manner as in Example 1. As a result, the agglomeration degree was found to be C rank, and the dissolving time was 35 minutes.

EXAMPLE 2

The operation was conducted in the same manner as in Example 1 except that the granulation time was changed from one minute to four minutes. The obtained TEDA was from 3.5 to 20 mesh (US Sieve Series which have openings 5660 to 840 micrometres across).

The agglomeration degree and the solubility of this product were measured, whereby the agglomeration degree was found to be A rank, and in the measurement of the solubility, the sample was readily dispersed and dissolved in the solution, and the dissolving time was 8 minutes.

EXAMPLE 3

The operation was conducted in the same manner as in Example 1 except that ethanol was used instead of methanol, and polyvinyl pyrrolidone was used instead of hydroxypropyl cellulose. The obtained TEDA was from 10 to 30 mesh.

The agglomeration degree and the solubility of this product were measured, whereby the agglomeration degree was found to be A rank, and in the measurement of the solubility, the sample was readily dispersed and dissolved in the solution, and the dissolving time was 5 minutes.

EXEMPLE 4

The operation was conducted in the same manner as in Example 1 except that 150 m$\ell$ of water was used instead of methanol, and 5 g of polyvinyl alcohol was used instead of hydroxypropyl cellulose. The obtained TEDA was from 10 to 30 mesh. However, the drying temperature was changed to 80°C.

The agglomeration degree and the solubility of the product were measured, whereby the agglomeration degree was found to be A rank, and in the measurement of the solubility, the sample was readily dispersed and dissolved in the solution, and the dissolving time was 8 minutes.

## Claims

1. Triethylenediamine which is a granular or particulate powder of at least 30 mesh (US Sieve Series which has openings 590 micrometres across) and thus all the particles have particle sizes in excess of 590 micrometres.

2. Triethylenediamine as claimed in Claim 1, characterised in that it contains from 0.5 to 10 parts by weight of an additive selected from the group consisting of celluloses, polyvinyl pyrrolidone, polyvinyl alcohol, glycols and polyethylene glycols, per 100 parts by weight of triethylenediamine.

3. Triethylenediamine as claimed in Claim 2, characterised in that the content of the additive is from 1 to 5 parts by weight per 100 parts by weight of triethylenediamine.

4. Triethylenediamine as claimed in Claim 1, 2 or 3 characterised in that the said powder is of from 10 to 15 mesh (US Sieve Series which have openings 2000 to 1190 to 1410 micrometres across).

5. A process for producing granular or particulate triethylenediamine, which comprises mixing from 3 to 20 parts by weight of water or an organic solvent and from 0.5 to 10 parts by weight of an additive to 100 parts by weight of powdery triethylenediamine, followed by granulation and drying.

6. A process as claimed in Claim 5, characterised in that the additive is selected from the group consisting of celluloses, polyvinyl pyrrolidone, polyvinyl alcohol, glycols and polyethylene glycols.

7. A process as claimed in Claim 5 or Claim 6 characterised in that the additive is mixed in an amount of from 1 to 5 parts by weight per 100 parts by weight of the powdery triethylenediamine.

8.  A process as claimed in Claim 5, 6 or 7 characterised in that the organic solvent is an alcohol.

9.  A process as claimed in Claim 8 characterised in that the organic solvent is methanol, ethanol or propanol.

10. A process as claimed in Claim 5, 6, 7, 8 or 9 characterised in that the said water or organic solvent is mixed in an amount of from 5 to 10 parts by weight per 100 parts by weight of powdery triethylenediamine.

**European Patent Office**

# EUROPEAN SEARCH REPORT

Application Number

EP 91 30 9394

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | DE-A-3 115 880 (AIR PRODUCTS AND CHEMICALS) <br><br> * abstract * <br> * page 7, paragraph 2 - page 15, last paragraph * <br><br> --- | 2,3,5-7, 10 | B01J2/30 <br> C07D487/08 |
| A | EP-A-0 209 720 (AIR PRODUCTS AND CHEMICALS) <br><br> * abstract * <br> * page 2, line 11 - page 3, line 9 * <br> * page 4, line 5 - page 6, line 29 * <br> --- | 2,3,5-7, 10 | |
| A | EP-A-0 149 186 (TOYO SODA MANUFACTURING CO.) <br> * the whole document * <br> --- | 1,5 | |
| A | GB-A-1 128 192 (ORGANON LABORATORIES LIMITED) <br> * page 1, right column, line 70 - page 4, left column, line 49 * <br> * claims 1-11 * | 2,3,5-10 | |
| | ----- | | **TECHNICAL FIELDS SEARCHED (Int. Cl.5 )** <br><br> B01J <br> C07D |

The present search report has been drawn up for all claims

| Place of search | Date of completion of the search | Examiner |
|---|---|---|
| THE HAGUE | 16 JANUARY 1992 | STEVNSBORG N. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)